# EUROPEAN PATENT APPLICATION

(11) **EP 2 597 097 A1**
(43) Date of publication of application: **29.05.2013**
(21) Application number: 11809940.7
(22) Date of filing: 19.07.2011
(51) Int. Cl.: C07D 487/04, C07D 401/06, C07D 401/14, C07D 209/82, C07D 209/88, A61K 31/496, A61K 31/498, A61K 31/5517, A61K 31/454, A61K 31/437, A61K 9/48, A61K 9/20, A61K 9/08, A61P 31/12

(54) **SUBSTITUTED INDOLES, ANTIVIRAL ACTIVE COMPONENT, METHOD FOR PRODUCING AND USING SAME**

(30) Priority: 23.07.2010 RU 2010130863
(71) Applicant: Alla Chem, LLC., Carson City NV 89701 (US); Limited Liability Company "MIP-11", Moskovskaya obl. 141400 (RU)
(72) Inventor: BICHKO, Vadim Vasilievich, San Diego CA 92129 (US); MITKIN, Oleg Dmitrievich, Moskovskaya obl. 141400 (RU); IVACHTCHENKO, Alexandre Vasilievich, Encinitas, CA 92024 (US)
(74) Representative: Henrion, Oliver
(86) International application number: PCT/RU2011/000533
(87) International publication number: WO 2012/011847

(57) **Abstract**

The invention relates to novel antiviral active components of the general formula 1, pharmaceutical composition, antiviral medicament, method for prophylaxis and treatment of viral diseases, particularly caused by hepatisis C viruses (HCV). In the general formula 1 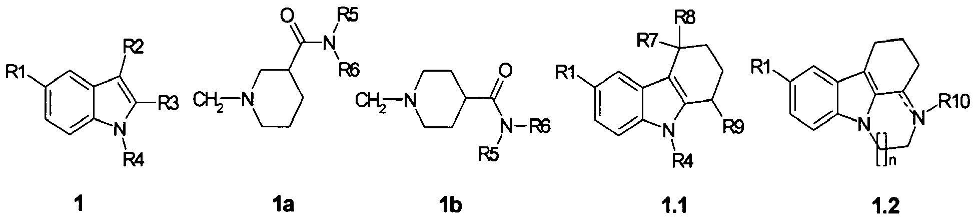 **R1** represents hydrogen, optionally substituted C₁-C₄ alkyl, C₆ cycloalkyl, aryl, ethoxycarbonyl, nitro group; **R2** represents hydrogen; **R3** represents *N*-mono-or *N,N*-disubstituted 1-methylene-piperidine-3-carboxamide of the general formula **1a** or *N*-mono- or *N,N*-disubstituted 1-methylene-piperidine-4-carboxamide of the general formula **1b; R4** represents hydrogen, optionally substituted C₁-C₃ alkyl; or **R2** and **R3** together with the C-atoms they are attached to form substituted ,3,4,9-tetrahydro-1*H*-carbazole of the general formula **1.1,** or **R2, R3,** and **R4** together with the atoms they are attached to form substituted azaheterocycles of the eneral formula **1.2; R5** and **R6** optionally identical represent hydrogen, optionally substituted C₁-C₃ alkyl or C₃-C₆ cydoalkyl, or **R5** and **R6** together with the N-atom they are attached to form optionally substituted 5- or 6- membered azaheterocyclyl comprising one or two N-atoms and optionally condensed with benzene ring;
**R7** and **R8** represent hydrogen or **R7** and **R8** together with the C-atom they are attached to form C=O group; **R9** represents azaheterocyclyl comprising at least one **N-atom,** unsubstituted formamide, or phenyl substituted with ethoxycarbonyl or nitro group; **R10** represents hydrogen, optionally substituted C₁-C₃ alkyl, substituted acetyl; n = 1 or 2; solid line accompanied by the dotted line, i.e. ( 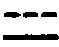 ), represents single or double C-C bond.

## Description

### Field of the invention

The present invention relates to novel antiviral component, pharmaceutical composition, antiviral medicament, method for prophylaxis and treatment of viral diseases, particularly caused by hepatitis C virus (HCV).

### Background of the invention

Virus infections may cause a great number of diseases that create a serious threat for health and existence of mankind. For the last 20 years no less than 30 essentially new infectious agents have been discovered such as: HIV, viral hepatitises, acute and long-lasting diarrhea, hemorrhagic fever (Ebola, Venezuelan, Brazilian, Rift valleys) [a) Lednicky J.A., Rayner J.O. Uncommon respiratory pathogens. Curr. Opin. Pulm. Med. 2006, 12(3), 235-239. b) Hayden F.G. Respiratory viral threats. Curr. Opin. Infect. Dis. 2006, 19(2), 169-178]. In particular, special anxiety is caused by the risk of infection by so named avian influenza. [a) Liu J.P. Avian influenza--a pandemic waiting to happen? J. Microbiol. Immunol. Infect. 2006, 39(1), 4-10. b) Henter J.I.; Chow C.B.; Leung C.W, Lau Y.L. Cytotoxic therapy for severe avian influenza A (H5N1) infection. Lancet. 2006 367(9513), 870-873. Review]. According to statistical data 60-65% of epidemic infections have viral ethiology. Because of the complexity of interaction in triad "virus - host's organism - drug", most of modern antiviral drugs display side effects in the course of therapy and form resistant virus strains [Jain R., Clark N.M., Diaz-Linares M., Grim S.A. Limitations of current antiretroviral agents and opportunities for development. Curr. Pharm. Des. 2006, 12(9), 1065-1074.]. At present, the number of antiviral drugs that could be used in clinical practice is extremely limited - only 43 low molecular weight substances [http://integrity.prous.com/integrity], that is far from covering requirements of prophylaxis and treatment of virus diseases. Moreover, there are a lot of virus infections causing diseases for treatment of which there are no chematherapeutic agents. This is particularly true, for example, for the diseases caused by viruses of papilloma, adenoviruses, herpes-6, variola, syndrome SARS, hemorrhagic fevers, fever of the Western Nile, avian influenza and so on [De Clercq E. Recent highlights in the development of new antiviral drugs. Curr Opin Microbiol. 2005, 8(5), 552-560].

Therefore, the development of novel antiviral medicaments, particularly exhibiting novel mechanism of antiviral action, high activity and low toxicity are of great importance now.

International applications WO 2009039246 A2 and WO 2009039248 A2 were published, in which novel antiviral active ingredients, which are Clemizole I and its analogs, for example, benzimidazole II, intended for prophylaxis and treatment of viral diseases caused by hepatitis C virus (HCV) were described.

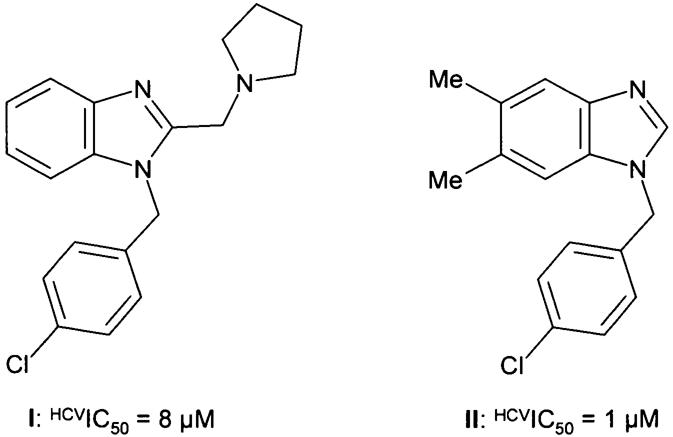

However, many substituted indoles exhibiting activity towards HCV have not been known so far.

Searching for highly effective antiviral medicaments is still one of the main directions in the development of novel pharmacological remedies for treatment of wide and various types of viral infections.

### Disclosure of the invention

In the context of the invention, the terms are generally defined as follows: **"Azaheterocycle"** means an aromatic or saturated mono- or poly- cyclic system, comprising at least one nitrogen atom in the cycle. Azaheterocycle may have one or more "cyclic system" substituents.

**"Active component"** (drug-substance) means a physiologically active compound of synthetic or other origin (biotechnological, vegetable, animal, microbe and so on), exhibiting pharmacological activity and being an active component of pharmaceutical composition, employing in production and preparation of medicaments.

"Alkyl" means an aliphatic hydrocarbon straight or branched chain with 1-12 carbon atoms. Branched means alkyl chain with one or more "lower alkyl" substituents. Alkyl group may have one or more substituents of the same or different structure ("alkyl substituent") including halogen, alkenyloxy, cycloalkyl, aryl, heteroaryl, heterocyclyl, aroyl, cyano, hydroxy, alkoxy, carboxy, alkynyloxy, aralkoxy, aryloxy, aryloxycarbonyl, alkylthio, heteroarylthio, aralkylthio, arylsulfonyl, alkylsulfonylheteroaralkyloxy, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, Rₖ^{a}Rₖ₊₁^{a}NC(=S)-, Rₖ^{a}Rₖ₊₁^{a}NSO₂, where Rₖ^{a} and Rₖ₊₁^{a} independently of each other represent "amino group substituents", for example, hydrogen, alkyl, aryl, aralkyl, heteroaralkyl, heterocyclyl or heteroaryl, or Rₖ^{a} and Rₖ₊₁^{a} together with the N-atom they are attached to form through Rₖ^{a} and Rₖ₊₁^{a} 4 - 7-membered heterocyclyl or heterocyclenyl. Methyl, trifluoromethyl, cyclopropylmethyl, cyclopentylmethyl, ethyl, n-propyl, *iso*-propyl, *n*-butyl, *tert.*-butyl, n-pentyl, 3-pentyl, methoxyethyl, carboxymethyl, methoxycarbonylmethyl, ethoxycarbonylmethyl, benzyloxycarbonylmethyl, methoxycarbonylmethyl and pyridylmethyloxycarbonylmethyl are the preferred alkyl groups. The preferred "alkyl substituents" are cycloalkyl, aryl, heteroaryl, heterocyclyl, hydroxy, alkoxy, alkoxycarbonyl, aralkoxy, aryloxy, alkylthio, heteroarylthio, aralkylthio, alkylsulfonyl, arylsulfonyl, alkoxycarbonyl, aralkoxycarbonyl, heteroaralkyloxycarbonyl or Rₖ^{a}Rₖ₊₁^{a}N-, Rₖ^{a}Rₖ₊₁^{a}NC(=O)-, annelated arylheterocyclenyl, annelated arylheterocyclyl.

**"Amino group"** means Rₖ^{a}Rₖ₊₁^{a}N - group substituted or not substituted with "amino group substituent" Rₖ^{a} and Rₖ₊₁^{a}, the meanings of which are defined in this section, for example, amino (H₂N-), methylamino, diethylamino, pyrrolidino, morpholino, benzylamino or phenethylamino.

**"Aryl"** means aromatic mono- or poly- cyclic system with 6 - 14 carbon atoms, predominantly 6-10 carbon atoms. Aryl may have one or more "cyclic system substituents" of the same or different structure. Phenyl, substituted phenyl, naphthyl, or substituted naphthyl are the representatives of aryl groups. Aryl could be annelated with saturated cyclic system or heterocycle.

**"Hydrate"** means stoichiometric or nonstoichiometric compositions of compounds or their salts with water.

**"Substituent"** means a chemical radical attached to a scaffold (fragment), for example, "alkyl substituent", "amino group substituent", "carbamoyl substituent", and "cyclic system substituent".

**"Amino group substituent"** means a substituent attached to amino group. Amino group substituent represents hydrogen, alkyl, cycloalkyl, aryl, heteroaryl, heterocyclyl, acyl, aroyl, alkylsulfonyl, arylsulfonyl, heteroarylsulfonyl, alkylaminocarbonyl, arylaminocarbonyl, heteroarylaminocarbonyl, heterocyclylaminocarbonyl, alkylaminothiocarbonyl, arylaminothiocarbonyl, heteroarylaminothiocarbonyl, heterocyclylaminothiocarbonyl, annelated heteroarylcycloalkenyl, annelated heteroarylcycloalkyl, annelated heteroarylheterocyclenyl, annelated heteroarylheterocyclyl, annelated arylcycloalkenyl, annelated arylcycloalkyl, annelated arylheterocyclenyl, annelated arylheterocyclyl, alkoxycarbonylalkyl, aralkoxycarbonylalkyl, heteroaralkyloxycarbonylalkyl.

**"Cyclic system substituent"** means a substituent attached to an aromatic or saturated cyclic system selected from hydrogen, alkyl, alkenyl, alkynyl, aryl, heteroaryl, aralkyl, heteroaralkyl, hydroxy, hydroxyalkyl, amino, aminoalkyl, alkoxy, aryloxy, acyl, aroyl, halogen, nitro, cyano, carboxy, alkoxycarbonyl, aryloxycarbonyl, aralkoxycarbonyl, alkyloxyalkyl, aryloxyalkyl, heterocyclyloxyalkyl, arylalkyloxyalkyl, heterocyclylalkyloxyalkyl, alkylsulfonyl, arylsulfonyl, heterocyclylsulfonyl, alkylfulfinyl, arylsulfinyl, heterocyclylsulfinyl, alkylthio, arylthio, heterocyclylthio, alkylsulfonylalkyl, arylsulfonylalkyl, heterocyclylsulfonylalkyl, alkylsulfinylalkyl, arylsulfinylalkyl, heterocyclylsulfinylalkyl, alkylthioalkyl, arylthioalkyl, heterocyclylthioalkyl, arylalkylsulfonylalkyl, heterocyclylalkylsulfonylalkyl, arylalkylthioalkyl, heterocyclylalkylthioalkyl, cycloalkyl, cycloalkenyl, heterocyclyl, heterocyclenyl, amidino, Rₖ^{a}Rₖ₊₁^{a}N-, Rₖ^{a}N=, Rₖ^{a}Rₖ₊₁^{a}N-alkyl, Rₖ^{a}Rₖ₊₁^{a}NC(=O)- or Rₖ^{a}Rₖ₊₁^{a}NSO₂-, where Rₖ^{a} and Rₖ₊₁^{a} independently of each other represent "amino group substituents", the meanings of which are defined in this section, for example, hydrogen, optionally substituted alkyl, optionally substituted aryl, optionally substituted aralkyl, optionally substituted heteroaralkyl or Rₖ^{a}Rₖ₊₁^{a}N-substituent in which Rₖ^{a} could be acyl or aroyl, the meaning of Rₖ₊₁^{a} is defined above, or "cyclic system substituents" are Rₖ^{a}Rₖ₊₁^{a}NC(=O)- or Rₖ^{a}Rₖ₊₁^{a}NSO₂- ,where Rₖ^{a} and Rₖ₊₁^{a} together with the N-atom they are attached to through Rₖ^{a} and Rₖ₊₁^{a} form 4-7-membered heterocyclyl or hetrocyclenyl.

**"Substituted amino group"** means Rₖ^{a}Rₖ₊₁^{a}N - group in which Rₖ^{a} and Rₖ₊₁^{a} represent amino group substituents, the meanings of which are defined in this section.

**"Substituted acetyl"** means RCH₂C(O)-group in which substituent R represents alkyl, amino group, aryl, heteroaryl, heterocyclyl, cycloalkyl.

**"Medicament"** - is a compound (or a mixture of compounds in the form of pharmaceutical composition) in the form of tablets, capsules, injections, ointments and other ready forms intended for restoration, improvement or modification of physiological functions at humans and animals, and also for treatment and prophylaxis of diseases, diagnostics, anesthesia, contraception, cosmetology and others.

**"Optionally substituted"** means a radical without substituents or a radical comprising one or more substituents.

**"Lower alkyl"** means a straight or branched alkyl with 1-4 carbon atoms. **"Therapeutic kit"** is a simultaneously administered combination of two or more drug substances with different mechanism of pharmacological action and aimed at different biotargets taking part in pathogenesis of the disease.

**"Pharmaceutical composition"** means a composition comprising a compound of general formula 1 and at least one of components selected from the group consisting of pharmaceutically acceptable and pharmacologicaly compatible fillers, solvents, diluents, auxiliary, distributing and sensing agents, delivery agents, such as preservatives, stabilizers, disintegrators, moisteners, emulsifiers, suspending agents, thickeners, sweeteners, flavouring agents, aromatizing agents, antibacterial agents, fungicides, lubricants, and prolonged delivery controllers, the choice and suitable proportions of which depend on the nature and way of administration and dosage. Examples of suitable suspending agents are ethoxylated isostearyl alcohol, polyoxyethene, sorbitol and sorbitol ether, microcrystalline cellulose, aluminum metahydroxide, bentonite, agar-agar and tragacant and their mixtures as well. Protection against action of microorganisms can be provided by various antibacterial and antifungal agents, such as, for example, parabens, chlorobutanole, sorbic acid, and similar compounds. Composition may also contain isotonic agents, such as, for example, sugar, sodium chloride, and similar compounds. Prolonged effect of composition may be achieved by agents slowing down absorption of active ingredient, for example, aluminum monostearate and gelatine. Examples of suitable carriers, solvents, diluents and delivery agents include water, ethanol, polyalcohols and their mixtures, natural oils (such as olive oil) and organic esters (such as ethyl oleate) for injections. Examples of fillers are lactose, milk-sugar, sodium citrate, calcium carbonate, calcium phosphate and the like. Examples of disintegrators and distributors are starch, alginic acid and its salts, and silicates. Examples of suitable lubricants are magnesium stearate, sodium lauryl sulfate, talc and polyethylene glycol of high molecular weight. Pharmaceutical composition for peroral, sublingval, transdermal, intramuscular, intravenous, subcutaneous, local or rectal administration of active ingredient, alone or in combination with another active compound may be administered to humans and animals in standard administration form, or in mixture with traditional pharmaceutical carriers. Suitable standard administration forms include peroral forms such as tablets, gelatin capsules, pills, powders, granules, chewing-gums and peroral solutions or suspensions, sublingval and transbuccal administration forms; aerosols; implants; local, transdermal, subcutaneous, intramuscular, intravenous, intranasal or intraocular forms of introduction and rectal administration forms.

**"Pharmaceutically acceptable salt"** means a relatively nontoxic both organic and inorganic salts of acids and bases disclosed in this invention. Salts could be prepared ***in situ*** in processes of synthesis, isolation or purification of compounds or be prepared specially. In particular, bases salts could be prepared starting from purified base of disclosed compound and suitable organic or mineral acid. Examples of salts prepared in this manner include hydrochlorides, hydrobromides, sulfates, bisulfates, phosphates, nitrates, acetates, oxalates, valeriates, oleates, palmitates, stearates, laurates, borates, benzoates, lactates, p-toluenesulfonates, citrates, maleates, fumarates, succinates, tartrates, methane sulphonates, malonates, salicylates, propionates, ethane sulphonates, benzene sulfonates, sulfamates and the like (Detailed description of properties of such salts is given in: Berge S.M., et al., "Pharmaceutical Salts" J.Pharm.Sci., 1977, 66: 1-19). Salts of disclosed acids may be also prepared by interaction of purified acids specifically with suitable base; moreover, metal salts and amine salts may be synthesized too. Metal salts are salts of sodium, potassium, calcium, barium, magnesium, zink, lithium and aluminum, sodium and potassium salts being preferred. Suitable inorganic bases from which metal salts can be prepared are sodium hydroxide, carbonate, bicarbonate and hydride; potassium hydroxide, carbonate and bicarbonate, lithium hydroxide, calcium hydroxide, magnesium hydroxide, zinc hydroxide. Organic bases suitable for preparation of disclosed acid salts are amines and amino acids of sufficient basicity to produce stable salt suitable for medical purposes use (in particular, they are to have low toxicity). Such amines include ammonia, methylamine, dimethylamine, trimethylamine, ethylamine, diethylamine, triethylamine, benzylamine, dibenzylamine, dicyclohexylamine, piperazine, ethylpiperidine, tris(hydroxymethyl)aminomethane and the like. Besides, salts can be prepared using some tetraalkylammonium hydroxides, such as holine, tetramethylammonium, tetraethylammonium, and the like. Aminoacids may be selected from main aminoacids - lysine, ornithine and agrinine. **"Cycloalkyl"** both as itself and as a part of other substituent means saturated mono- or poly- cyclic system with 3 - 10 carbon atoms, more preferably, C₃-C₆ cycloalkyl. Cycloalkyl may have one or more "cyclyc system substituents" of the same or different structure. Cyclopropyl, cyclobutyl, cyclopentyl, cyclohexyl, cycloheptyl, decaline, adamant-1-yl and so on are the representatives of cycloalkyl groups. Cycloalkyl may be annelated with aromatic cycle or heterocycle. The preferred "cyclic system substituents" are alkyl, aralkoxy, hydroxy or Rₖ^{a}Rₖ₊₁^{a}N. The subject of the present invention is novel antiviral component, which is substituted indole of the general formula **1** and pharmaceutically acceptable salts thereof 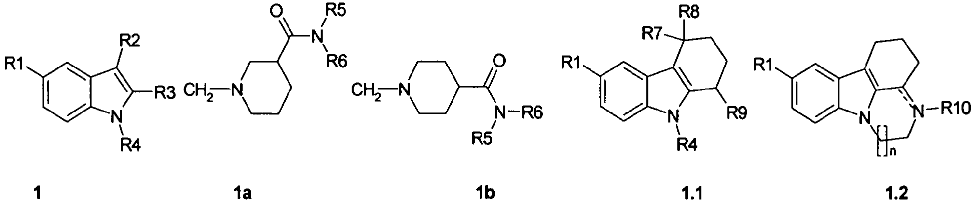 wherein:
**R1** represents hydrogen, optionally substituted C₁-C₄alkyl, cycloalkyl, aryl, ethoxycarbonyl, nitro group;
**R2** represents hydrogen;
**R3** represents *N*-mono- or *N*,*N*-disubstituted 1-methylene-piperidine-3-carboxamide of the general formula **1a** or *N*-mono- or *N,N*-disubstituted 1-methylene-piperidine-4-carboxamide of the general formula **1b**;
**R4** is hydrogen, optionally substituted C₂-C₃ alkyl, or -CH₂-**R12** group, wherein **R12** is hydrogen or phenyl optionally substituted with C₁-C₄ alkyl or halogen; or
**R2, R3,** and **R4** together with the atoms they are attached to form two substituted annelated with pyrrole ring and with each other 6- or 6- and 7-membered cycles representing azaheterocycles of the general formula **1.2;**
**R5** and **R6** optionally identical, represent hydrogen, optionally substituted C₁-C₃alkyl or cycloalkyl or
**R5** and **R6** together with the N-atom they are attached to form optionally substituted 5- or 6- membered azaheterocyclyl comprising one or two nitrogen atoms and optionally condensed with benzene ring, or form group 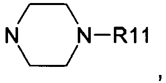
wherein:
**R11** is hydrogen, C₁-C₆ alkyl,
C₃-C₆ cycloalkyl;
**R10** represents hydrogen, optionally substituted C₁-C₃alkyl, optionally substituted acetyl,
n = 1 or 2;
solid line accompanied by the dotted line, i.e. 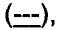 , represents single or double C-C-bond.

The preferred antiviral active component is substituted amide of 1-[(1H-indol-2-yl)methyl]piperidine-4-carboxylic acid of the general formula 1.3.1 or substituted amide of 1-[(1H-indol-2-yl)methyl]piperidine-3-carboxylic acid of the general formula 1.3.2 and pharmaceutically acceptable salts thereof 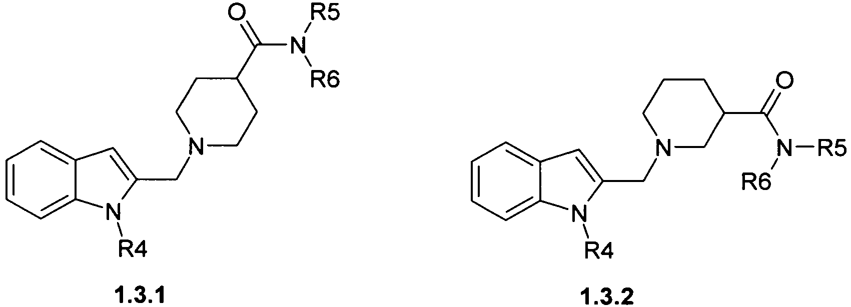 wherein:
**R4, R5** and **R6** have the above meanings.

The more preferred antiviral active component is the antiviral active component representing substituted amide of 1-[(1H-indol-2-yl)methyl]piperidine-4-carboxylic acid of the general formula **1.3.1.1** and pharmaceutically acceptable salts thereof 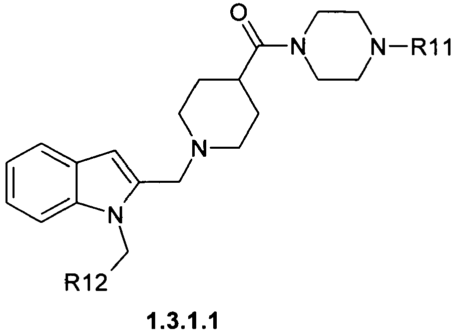 wherein:
**R11** represents C₁-C₃alkyl;
**R12** represents hydrogen or phenyl optionally substituted with halogen or C₁-C₃alkyl.

The more preferred antiviral active component is also the unknown before 2-(8-isopropyl-1,2,3a,4,5,6-hexahydro-3H-pyrazino[3,2,1-Jk]carbazol-3-yl)acetamide and its hydrochloride of formula **1.2(1)·HCl**

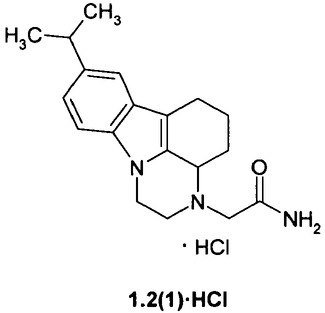

The more preferred antiviral active component is the antiviral active component exhibiting antiviral activity towards HCV, representing substituted indole of the general formula 1, wherein **R2** and R3 together with the carbon atoms they are attached to form substituted 6-membered saturated and annelated with pyrrole ring cycle representing 2,3,4,9-tetrahydro-1*H*-carbazole of the general formula 1.1 and pharmaceutically acceptable salt thereof 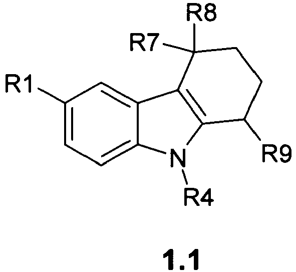 wherein:
**R₁** is methyl, ethoxycarbonyl, phenyl, nitro group,
**R4** is hydrogen, methyl, C₂-C₃ alkyl optionally substituted with N-benzylamine;
**R7** and **R8** are hydrogen, or **R7** and **R8** together with the C-atom they are attached to form C=O group;
**R9** is azaheterocyclyl comprising at least one N-atom, unsubstituted formamide, or N-phenylamine substituted with ethoxycarbonyl or nitro group.

The more preferred antiviral active component is the antiviral active component exhibiting antiviral activity towards HCV, representing substituted indole of the general formulas **1, 1.2, 1.3, 1.3.1, 1.3.1.1, 1.3.2.1** or pharmaceutically acceptable salts thereof.

The subject of the present invention is also a pharmaceutical composition exhibiting antiviral activity and comprising pharmaceutically effective amount of antiviral active component representing at least one substituted indole of the general formulas **1, 1.2, 1.3, 1.3.1, 1.3.1.1, 1.3.2.1** or pharmaceutically acceptable salts thereof.

The more preferable composition is the pharmaceutical composition exhibiting antiviral activity towards hepatitis C virus (HCV) comprising as an active component at least one substituted indole of the general formulas **1, 1.1, 1.2, 1.3, 1.3.1, 1.3.1.1, 1.3.2.1** or pharmaceutically acceptable salts thereof.

Pharmaceutical compositions may include pharmaceutically acceptable excipients. Pharmaceutically acceptable excipients mean diluents, auxiliary agents and/or carriers employing in the sphere of pharmaceutics. According to the invention the pharmaceutical composition in addition to substituted indole of the general formulas **1, 1.1, 1.2, 1.3, 1.3.1, 1.3.1.1, 1.3.2.1** or pharmaceutically acceptable salt thereof may include other active components, provided that they do not give rise to undesirable side-effects, for example, allergic reactions.

According to the present invention the pharmaceutical compositions can be mixed on demand with other components for preparation of various forms suitable for applicational in clinical use, including traditional pharmaceutical carriers; for example peroral forms (such as tablets, gelatin capsuls, pills, sollutions or suspensions); forms for injections (such as sollutions or suspensions for injections or dry powder for injections, demanding addition of water for injections before use); local forms (such as creams or sollutions).

According to the present invention the carriers used in pharmaceutical compositions represent carriers which are applied in the sphere of pharmaceutics for preparation of commonly used forms including: binding agents, greasing agents, disintegrators, solvents, diluents, stabilizers, suspending agents, colorless agents, taste flavors are used for peroral forms; antiseptic agents, solubilizers, stabilizers are used in the forms for injections; base materials, diluents, greasing agents, antiseptic agents are used in local forms.

The subject of the present invention is also a method for preparation of pharmaceutical composition.

The object in view is achieved by mixing, at least one antiviral active component representing substituted indole of the general formulas **1, 1.1, 1.2, 1.3, 1.3.1, 1.3.1.1, 1.3.2.1** or pharmaceutically acceptable salts thereof with inert filler and/or solvent.

The subject of the present invention is also a medicament intended for treatment of viral diseases comprising novel antiviral component which is at least one substituted indole of the general formulas 1, **1.2, 1.3, 1.3.1, 1.3.1.1, 1.3.2.1** or pharmaceutically acceptable salt thereof, or novel pharmaceutical composition including that active component in the form of tablets, sheaths or injections placed in pharmaceutically acceptable packing.

According to the invention the more preferable medicament is the medicament intended for treatment diseases caused by HCV viruses comprising novel antiviral component which is at least one substituted indole of the general formulas **1, 1.1, 1.2, 1.3, 1.3.1, 1.3.1.1, 1.3.2.1** or pharmaceutically acceptable salt thereof, or novel pharmaceutical composition including that active component in the form of tablets, capsuls or injections placed in pharmaceutically acceptable packing.

Medicaments could be administered perorally or parenterally, for example, intravenously, subcutaneously, intraperitoneally or locally. The clinical dosage of the pharmaceutical composition or medicament comprising antiviral active component of the general formulas **1, 1.1, 1.2, 1.3, 1.3.1, 1.3.1.1, 1.3.2.1** could be corrected depending on: therapeutic efficiency and bioavailability of the active ingredients in organism, rate of their exchange and deducing from organism, and also depending on the age, sex and the severity of the patient's symptoms; the daily dosage for adults falls within the range of about 10 to about 500 mg of the active ingredient, preferably of about 50 to about 300 mg. Therefore, according to the present invention in the process of preparation of a medicament from the pharmaceutical composition as units of dosage it is necessary to keep in mind the above effective dosage, so that each unit of dosage should contain of about 10 to about 500 mg of active component of the general formulas **1, 1.1, 1.2, 1.3, 1.3.1, 1.3.1.1, 1.3.2.1** preferably 50 ~ 300 mg. In accordance with the recommendation of physician or pharmacist the above dosage can be taken several times during the definite time intervals (preferably - from one to six times).

The subject of the present invention is also a therapeutic cocktail for prophylaxis and treatment of viral diseases, among them diseases caused by hepatitis C viruses, including novel active component or novel pharmaceutical composition comprising, at least one substituted indole of the general formulas **1, 1.1, 1.2, 1.3, 1.3.1, 1.3.1.1, 1.3.2.1** or pharmaceutically acceptable salts thereof.

Therapeutic cocktails for prophylaxis and treatment of hepatitis C along with the medicament disclosed in the invention, may include: inosine-5-monophosphate dehydrogenase inhibitors, for example, Ribavirin (licensed) and Ribamidil; inhibitors of hepatitis C NS3 protease, for example, Telaprevir, Ciluprevir and SCH-503034; inhibitors of RNA-polymerase NS5B, for example, XTL-2125; alpha-glucosidase inhibitors, for example, aminocarbohydrate Selgozivir; and also TLR-receptor agonists, hepatoprotectors, cyclosporines, various proteins (for example, interferons), antibodies, vaccines and so on.

The subject of the present invention is also method for prophylaxis and treatmemt of viral diseases at animals and humans.

According to the invention method for prophylaxis and treatment of viral diseases at humans and animals, among them diseases caused by hepatitis C viruses, consists in introduction to warm-blooded animal or human novel medicament, or novel pharmaceutical composition or novel therapeutic cocktail, comprising novel antiviral active component of the general formulas **1, 1.1, 1.2, 1.3, 1.3.1, 1.3.1.1, 1.3.2.1** or pharmaceutically acceptable salt thereof.

The subject of the present invention is the unknown before substituted amides of 1-[(1H-indol-2-yl)methyl]piperidine-4-carboxylic acid of the general formula **1.3.1** and substituted amides of 1-[(1H-indol-2-yl)methyl]piperidine-3-carboxylic acid of the general formula **1.3.2** and pharmaceutically acceptable salts thereof 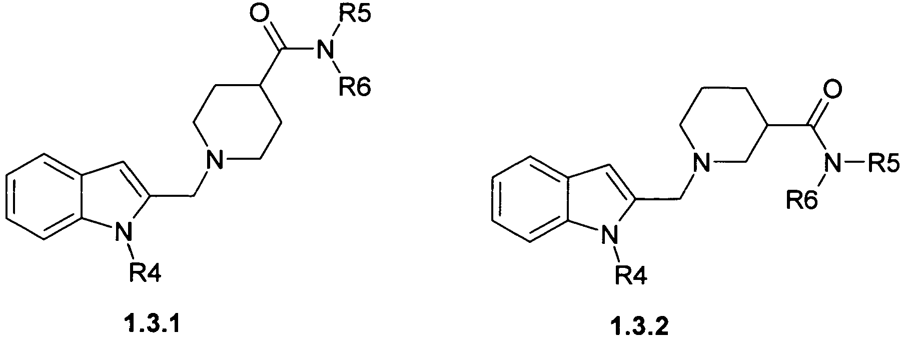 wherein:
**R4, R5** and **R6** have the above meanings.

The subject of the present invention are the unknown before substituted amides of 1-[(1H-indol-2-yl)methyl]piperidine-4-carboxylic acid of the general formula **1.3.1.1** and substituted amides of 1-[(1H-indol-2-yl)methyl]piperidine-3-carboxylic acid of the general formula **1.3.2.1** and pharmaceutically acceptable salts thereof. 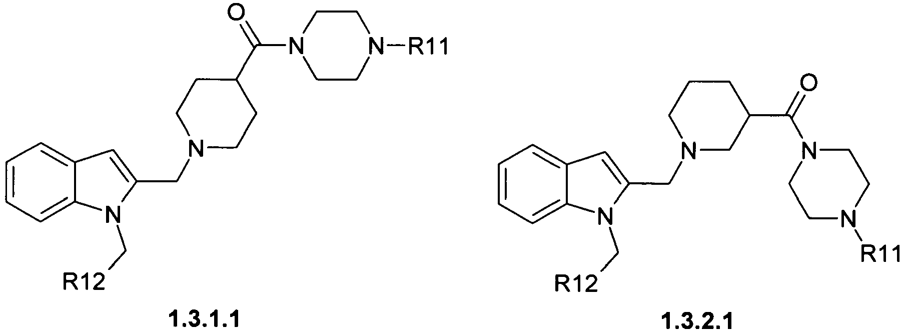 wherein:
**R11** represents C₁-C₃alkyl; **R12** represents hydrogen or phenyl optionally substituted with halogen or C₁-C₃alkyl.

The more preferable substituted amides are: {1-[1-(4-chlorobenzyl)-1*H*-indol-2-ylmethyl]-piperidin-3-yl}-(4-propylpiperazin-1-yl)-methanone **1.3.2.1(13),** [1-(1-methyl-1*H*-indol-2-ylmethyl)-piperidin-4-yl]-(4-propylpiperazin-1-yl)-methanone 1.3.1.1(14), [1-(1-methyl-1*H*-indol-2-ylmethyl)-piperidin-3-yl]-(4-propylpiperazin-1-yl)-methanone 1.3.2.1(14) and hydrochlorides thereof

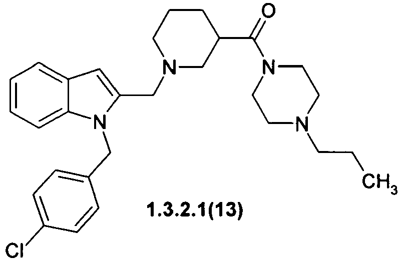

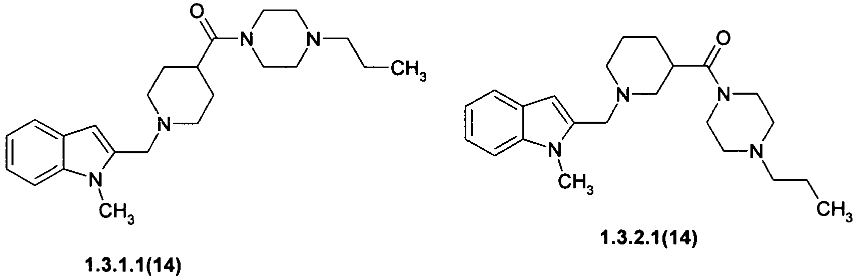

The subject of the present invention is also method for preparation of substituted amides of 1-(1H-indol-2-ylmethyl)-piperidine-carboxylic acids of the general formulas 1.3.1, 1.3.2 and pharmaceutically acceptable salts thereof which consists in interaction of substituted 1-(1H-indol-2-ylmethyl)-piperidine-carboxylic acids of the general formulas 2.1, 2.2 with amines 3. The best results were obtained if the reaction was carried out in the presence of 1,1'-carbonyldiimidazole. Pharmaceutically acceptable salts of amides **1.3.1, 1.3.2** were prepared by interaction of bases **1.3.1, 1.3.2** with the corresponding acids in the medium of suitable organic solvent. 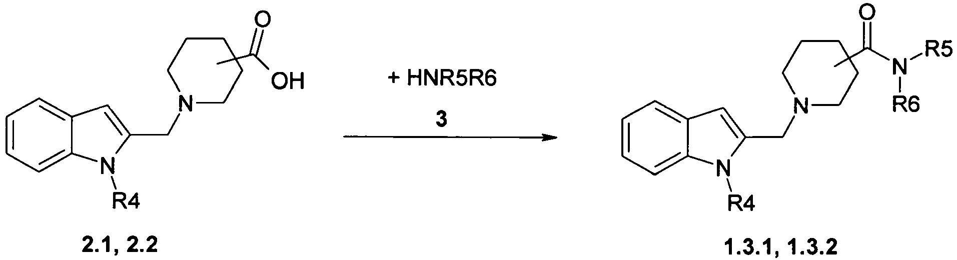 wherein:
**R4, R5** and **R6** have the above meanings.

The subject of the present invention is also method for preparation of 2-(8-isopropyl-1,2,3a,4,5,6-hexahydro-3H-pyrazino[3,2,1-Jk]carbazol-3-yl)acetamide hydrochloride of formula **1.2(1)·HCl** by hydrogenation of 9-[2-(dibenzylamino)ethyl]-6-isopropyl-3,4-dihydro-2H-carbazol-1 (9*H*)-one in the presence of 10% Pd/C with subsequent alkylation of obtained 2-(8-isopropyl-1,2,3a,4,5,6-hexahydro-3*H*-pyrazino[3,2,1*jk*]carbazole by chloroacetamide and transformation of the prepared base into its hydrochloride according to the following scheme:

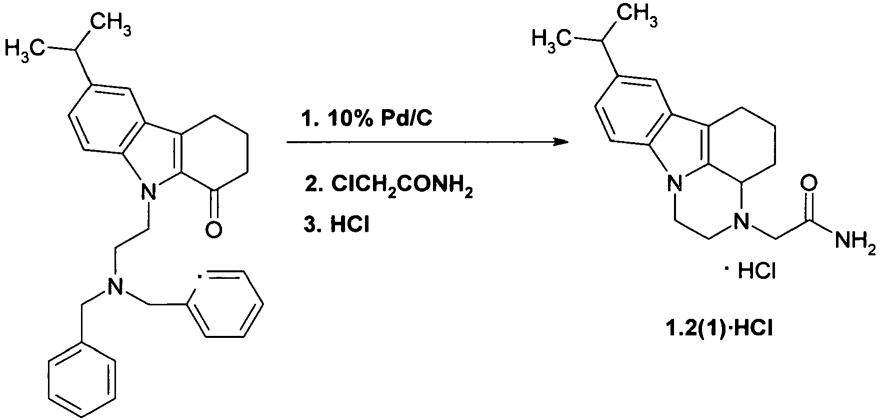

### Best Embodiment of the invention

The examples given below illustrate but not limit the scope of the invention.

**Example 1.** Determination of antiviral activity of compounds of the general formula **1** towards hepatitis C viruses.

Determination of antiviral activity of compounds of the general formulas **1, 1.1, 1.2, 1.3, 1.3.1, 1.3.1.1** towards HCV was carried out on Huh7 cell culture. Immune *in vitro* (ELISA - enzyme-linked immunosorbent assay) test ELISA towards HCV virus cor-antigen was used. By means of this test inhibition effectiveness of antigen production (and, consequently, virus replication) by tested compounds in cells infected by JFH-1 virus was measured. Used in this experiment Huh7 cell culture having been infected with HCV virus is capable to support full range of viral replication.

Recombinant JFH-1 HCV virus used in the experimemnt was prepared by means of trasfection of Huh7 cells by RNA JFH-1, prepared by transcription *in vitro.* JFH-1 DNA [Wakita, T. u ∂p. Production of Infectious Hepatitis C Virus in Tissue Culture from a Cloned Viral Genome. Nature Medicine 2005, 11:791-796.]) used as DNA matrix for transcription (NCBI, catalogue number AB047639, was synthesized chemically.

Huh7 cells were sowed in 96-well plates (3,0×10³ cells in every well) Huh-7 medium (50 µl per every well). 9 solutions of every tested compound in Huh-7 medium in the range of concentrations of 0.09-200 µM were prepared. In 4 h after cell sowing the initial solution was removed by aspiration, after that 50 µl of tested compound solution and 50 µl of JFH-1 HCV were added to every well. As a result, the final concentrations of the tested compounds were in the range of 0.045-100 µM. If required the step of infection was carried out in 24 h after addition of tested compounds. After incubation for 16 h viral medium was removed by aspiration, and the tested compounds were added to the cultures in initial concentration to final value of 200 µl. The cells and tested compounds were incubated additionally for 4 days at 37 °C in the atmosphere of air containing 5% CO₂. After the removal of nutrient solution the cells were fixed by addition of acetone/methanol (1:1) mixture in amount of 250 µl/well for 1 minute, washed three times with Phosphate Buffer (PBS buffer and then they were blocked by 10% FBS-PBS solution (150 µl/well)) for 1 h at room temperature. Each well was washed three times with PBS solution, incubated with mouse monoclonal antibodies to HCV cor-antigen (Affinity BioReagents, Catalogue: MA1-080), dissolved in FBS-PBS buffer (100 µl/well, prepared from the initial concentrate, diluted in the ratio 1:500 in 10% FBS- PBS), for 2 h at 37 °C. Each well was washed three times with PBS solution, incubated with antibodies specific to mouse immunoglobulins, conjugated with horseradish peroxidase (HRP, 100 mkl/well of solution prepared from the initial concentrate, diluted in ratio 1:2500 in 10% FBS- PBS) for 1 h at 37 °C. Each well was washed three times with PBS solution, then treated with OPD solution in amount of 100 µl/well (prepared by dilution of one OPD tablet in B 12 ml of citrate/phosphate buffer with addition of 5 µl of 30% H₂O₂) for 30 min in darkness at room temperature. The reaction was stopped by addition of 2N H₂SO₄ (100 µl/well), after that optical density at 490 nm was measured. IC₅₀ value representing the concentration of the tested compound at which virus replication is lowered twice was calculated for every tested compound using Xlfit program according to the method described in [Wakita, T. u ∂p. (2005). Production of Infectious Hepatitis C Virus in Tissue Culture from a Cloned Viral Genome. Nature Medicine; 11:791-796.].

The Table shows IC₅₀ values of substituted indoles of the general formulas **1, 1.1, 1.2, 1.3, 1.3.1, 1.3.1.1,** reflecting their ability to inhibit hepatitis C virus.

**Table.**

| Inhibition of hepatitis C virus (IC₅₀) by substituted indoles of the general formula 1 | | | | | | |
|---|---|---|---|---|---|---|
| Substituted indole | Formula | | | | | IC₅₀ (mM) |
| **I** Control | 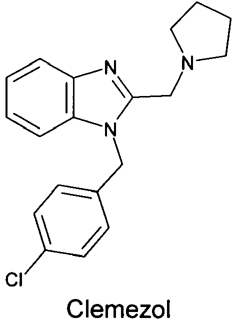 | | | | | 9.0 |

| Table. | | | | | | |
|---|---|---|---|---|---|---|
| 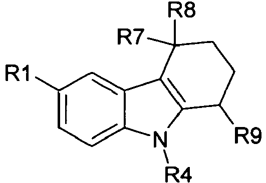 | | | | | | |

| Substituted indole | **R1-** | **R4-** | **R7-** | **R8-** | **R9-** | IC₅₀ (mM) |
|---|---|---|---|---|---|---|
| **1.1(1)** | 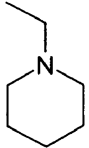 | 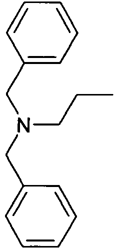 | =O | | H | 1.0 |
| **1.1(2)** | CH₃- | H | H | H | -NH₂ | 6.4 |
| **1.1(3)** | CH₃- | H | H | H | 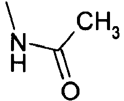 | 5.5 |
| **1.1(4)** | 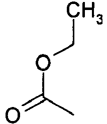 | H | H | H | 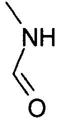 | 4.2 |
| **1.1(5)** | 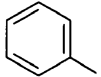 | H | H | H | 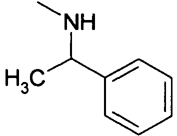 | 7.0 |

| Table. | | | | | | |
|---|---|---|---|---|---|---|
| **1.1(6)** | CH₃- | H | H | H | 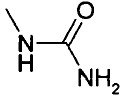 | 3.7 |
| **1.1(7)** | NO₂- | H | H | H | 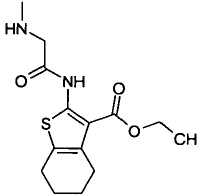 | 3.6 |
| **1.1(8)** | CH₃- | H | H | H | 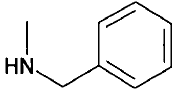 | 0.4 |
| **1.1(9)** | CH₃- | H | H | H | 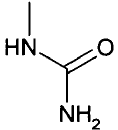 | 2.8 |
| **1.1(10)** | CH₃- | H | H | H | 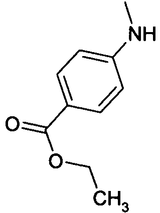 | <0.4 |
| **1.1(11)** | CH₃- | H | H | H | 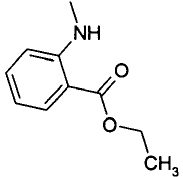 | 0.6 |
| **1.1(12)** | CH₃- | H | H | H | 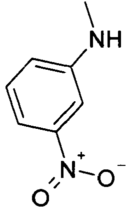 | <0.4 |
| **1.1(13)** | CH₃- | H | H | H | 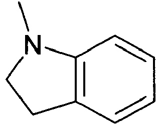 | 1.3 |
| 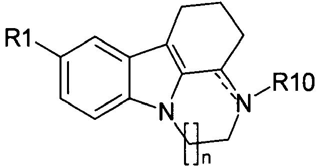 | | | | | | |

| Substituted indole | **R1-** | | | **R10-** | | IC₅₀ (mM) |
|---|---|---|---|---|---|---|
| **1.2(1)** | i-Pr | | | 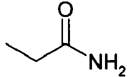 | | 1.7 |
| **1.2(2)** | i-Pr | | | H | | 4.1 |
| **1.2(3)** | H | | | 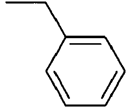 | | 5.1 |
| **1.2(4)** | CH₃- | | | 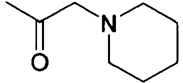 | | 4.0 |

| Table. | | | | | | |
|---|---|---|---|---|---|---|
| **1.2(5)** | t-Bu | | 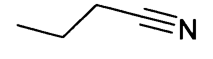 | | | 4.4 |
| **1.2(6)** | 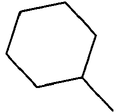 | | 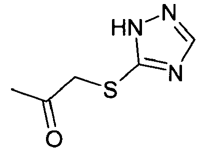 | | | 2.9 |
| **1.2(7)** | 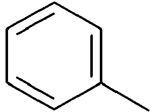 | | H | | | 0.7 |
| **1.2(8)** | 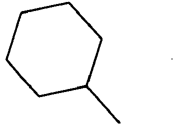 | | 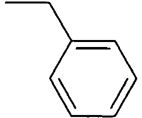 | | | 1.6 |
| 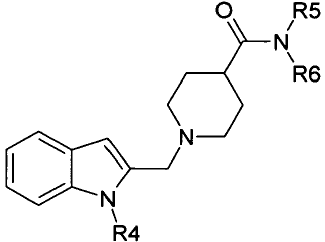 | | | | | | |

| Substituted indole | **R4-** | | **R5-** | **R6-** | | IC₅₀ (mM) |
|---|---|---|---|---|---|---|
| **1.3.1(1)** | 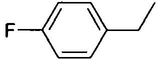 | | H | 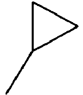 | | 3.9 |
| **1.3.1(2)** | 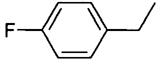 | | 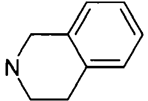 | | | 2.9 |

| Table. | | | | | | |
|---|---|---|---|---|---|---|
| **1.3.1(3)** | 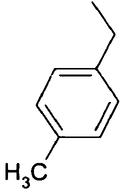 | CH₃- | | 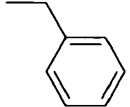 | | 6.4 |
| **1.3.1(4)** | 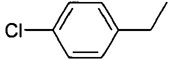 | 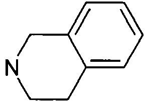 | | | | 5.4 |
| **1.3.1(5)** | 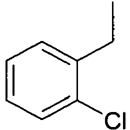 | H | | 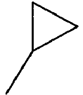 | | 6.2 |
| **1.3.1(6)** | 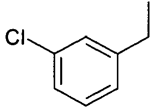 | H | | 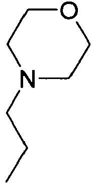 | | 2.4 |
| **1.3.1(7)** | 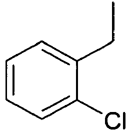 | H | | 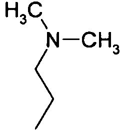 | | 0.8 |
| **1.3.1(8)** | 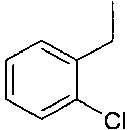 | H | | 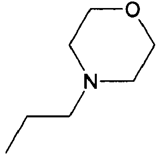 | | 3.3 |
| **1.3.1(9)** | 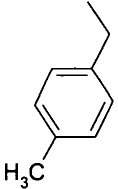 | H | | | 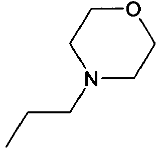 | 2.2 |
| **1.3.1(10)** | 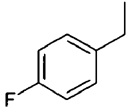 | H | | | 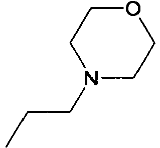 | 2.6 |
| **1.3.1(11)** | 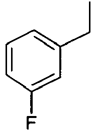 | H | | | 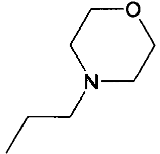 | 3.3 |
| **1.3.1(12)** | 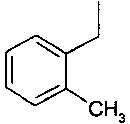 | H | | | 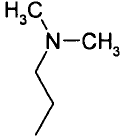 | 0.4 |
| **1.3.1(13)** | 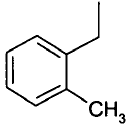 | H | | | 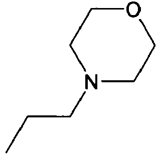 | 1.9 |
| **1.3.1(14)** | 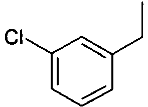 | H | | | 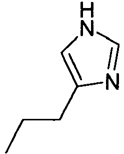 | 1.2 |
| **1.3.1(15)** | 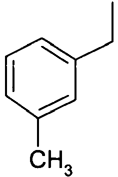 | H | | | 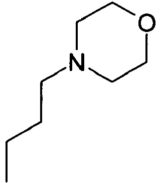 | 1.2 |
| **1.3.1(16)** | 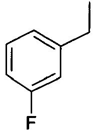 | H | | | 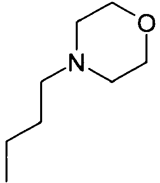 | 0.7 |
| **1.3.1(17)** | 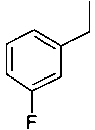 | H | | | 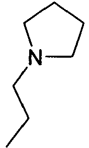 | 1.0 |
| **1.3.1(18) * 2 HCl** | CH₃- | H | | | 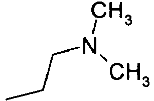 | 5.7 |
| **1.3.1(19) * 2 HCl** | CH₃- | H | | | 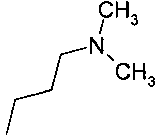 | 7.7 |
| 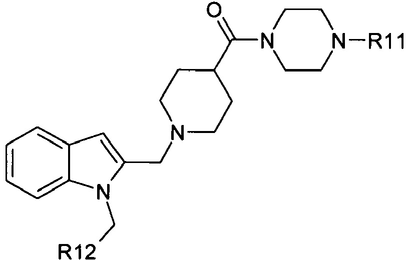 | | | | | | |

| Substituted indole | **R11-** | | | **R12-** | | IC₅₀ (mM) |
|---|---|---|---|---|---|---|
| **1.3.1.1(1)** | 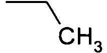 | | | 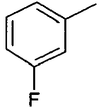 | | 0.8 |
| **1.3.1.1(2)** | 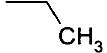 | | | 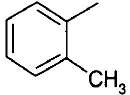 | | 0.3 |
| **1.3.1.1(3)** | 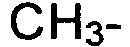 | | | 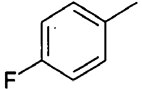 | | 0.9 |
| **1.3.1.1(4)** | 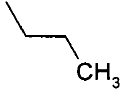 | | | 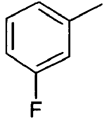 | | 1.4 |
| **1.3.1.1(6)** | CH₃- | | | 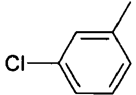 | | 0.6 |
| **1.3.1.1(7)** | 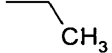 | | | 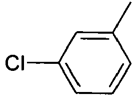 | | 0.4 |
| **1.3.1.1(8)** | CH₃- | | | 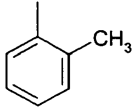 | | 0.7 |
| **1.3.1.1(9)** | 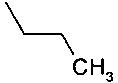 | | | 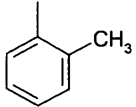 | | 0.5 |
| **1.3.1.1(10)** | 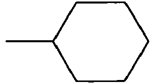 | | | 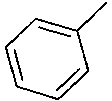 | | 0.5 |
| **1.3.1.1(11)** | CH₃- | | | 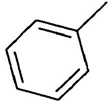 | | 1.2 |
| **1.3.1.1(12)** | CH₃- | | | 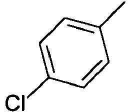 | | 0.5 |
| **1.3.1.1(13)** | 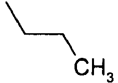 | | | 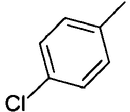 | | 0.3 |
| **1.3.1.1(14) * 2 HCl** | 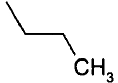 | | | CH₃- | | 2.1 |
| **1.3.1.1(15) * 2 HCl** | CH₃- | | | CH₃- | | 2.4 |
| 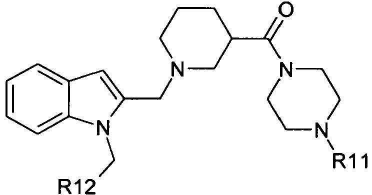 | | | | | | |

| Substituted indole | **R11-** | | | **R12-** | | IC₅₀ (mM) |
|---|---|---|---|---|---|---|
| **1.3.2.1(13) * 2 HCl** | 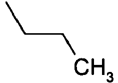 | | | 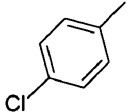 | | 0.4 |

Substituted indoles of the general formulas **1, 1.1, 1.2, 1.3, 1.3.1, 1.3.1.1, 1.3.2.1** exhibit high activity towards HCV (Table). As a rule, they are more active, then the known agents I and II [WO 2009039246 A2, WO 2009039248 A2]. For example, compounds **1.3.1.1(2)** and **1.3.1.1 (13)** have IC₅₀ = 0,3 mM, and compounds **1.1 (8), 1.3.1(2)** and **1.3.1.1(7)** have IC₅₀ = 0,4 mM.

**Example 2. 2-(8-Isopropyl-1,2,3a,4,5,6-hexahydro-3*H*-pyrazino[3,2,1-*jk*]carbazol-3-yl)-acetamide hydrochloride 1.2(1)·HCl.** Solution of 9-[2-(dibenzylamino)ethyl]-6-isopropyl-3,4-dihydro-2H-carbazol-1 (9*H*)-one (8.45 g, 18.8 mmol) in methanol (80 ml) was hydrogenated with 10 % Pd/C (0.5 g) in autoclave at 30 atm for 24 h at 70 °C. The solution was filtered through celit and evaporated in *vacuo.* The residue of obtained 2-(8-isopropyl-1,2,3a,4,5,6-hexahydro-3*H-*pyrazino[3,2,1-*jk*]carbazole was boiled with chloroacetamide (1.93 g, 20.6 mmol) and K₂CO₃ (3.88 g) in acetonitrile (50 ml) for 12 h. The solvent was distilled in *vacuo,* the residue was treated with water and extracted with CH₂Cl₂. Extract was dried over Na₂SO₄, evaporated and the product was recrystallized from ethanol. It gave 3.27 g (56 %) 2-(8-isopropyl-1,2,3a,4,5,6-hexahydro-3*H*-pyrazino[3,2,1-*jk*]carbazol-3-yl)-acetamide 1.2(1). LCMS (M+H)⁺ 312. ¹H NMR (CDCl₃, 400 MHz) δ 7.34 (s, 1H), 7.21 (d, *J* = 8.4 Hz, 1 H), 7.10 (dd, *J₁* = 8.4 Hz, *J₂* = 1.2 Hz, 1 H), 7.02 (br. s, 1 H), 5.50 (br.s, 1 H), 4.18 (dd, *J₁* = 11.6 Hz, *J₂* = 3.6 Hz, 1 H), 3.84 (dt, *J₁* = 11.6 Hz, *J₂* = 4.8 Hz, 1H), 3.59 (m, 1 H), 3.53 (d, *J* = 12.8 Hz, 1 H), 3.23 (dd, *J₁* = 12.4 Hz, *J₂* = 4.8 Hz, 1 H), 3.06 (m, 3H), 2.82 (dd, *J₁* = 11.6 Hz, *J₂* = 6.4 Hz, 1 H), 2.70 (m, 1 H), 2.22 (m, 2H), 1.86 (m, 1 H), 1.53 (m, 1 H), 1.32 (d, *J* = 6.8 Hz, 6H). 2-(8-Isopropyl-1,2,3a,4,5,6-hexahydro-3*H*-pyrazino[3,2,1-*jk*]carbazol-3-yl)-acetamide hydrochloride **1.2(1)·HCl** was precipitated by addition of 5 % excess of 3N HCl solution in dioxane to solution of base **1.2(1)** in CH₂Cl₂. ¹H NMR (DMSO-*d*₆, 400 MHz) δ 10.89 (br. s, 1 H), 8.16 (br. s, 1 H), 7.75 (br. s, 1 H), 7.34 (d, *J* = 8.0 Hz, 1 H), 7.31 (s, 1 H), 7.10 (d, *J* = 8.0 Hz, 1 H), 4.77 (br. m, 1 H), 4.46 (d, *J* = 6.4 Hz, 1 H), 4.25 (br. m, 1 H), 3.95 (br. m, 4H), 2.97 (d, *J* = 6.8 Hz, 1 H), 2.73 (br. d, *J* = 15.6 Hz, 1 H), 2.58 (br. m, 1H), 2.35 (br. m, 1H), 2.20 (br. m, 1H), 1.78 (br. m, 2H), 1.24 (d, J = 6.8 Hz, 6H).

**Example 3.** Amides of 1-(1H-indol-2-ylmethyl)piperidine-carboxylic acids of the general formulas **1.3.1, 1.3.2** (general method). 1,1'-Carbonyldiimidazole (1,95 g, 12 mmol) was added to suspension of acid **6** (10 mmol) in dry acetonitrile (45 ml). The mixture was stirred for 3 h at 70 °C (TLC control), cooled to room temperature, then the corresponding piperazine (12 mmol) was added, and stirring was continued at 70 °C for 12 h (LCMS control). After the reaction was completed the mixture was poured into water, precipitated solid was filtered off, washed with water several times and dried in *vacuo.* It gave amides **1.3.1, 1.3.2.** Dihydrochlorides of amides **1.3.1, 1.3.2** were prepared by addition of 5 % excess of 3 N **HCl** solution in dioxane to solution of amides in ether (5 g in 500 ml).

**1-Methyl-2-({4-[(4-propylpiperazin-1-yl)carbonyl]piperidin-1-yl}methyl)-1*H-***indole 1.3.1.1(14). Yield is 28 %. LCMS (M+H)⁺ 383. ¹H NMR (DMSO-*d*₆, 400 MHz) δ 11.29 (br. s, 1H), 10.80 (br. s, 1 H), 7.60 (d, *J* = 8.0 Hz, 1 H), 7.51 (d, *J* = 8.0 Hz, 1 H), 7.23 (m, 1 H), 7.09 (m, 1 H), 6.80 (s, 1 H), 4.55 (br. s, 2H), 4.39 (br. m, 1 H), 4.11 (br. m, 1 H), 3.85 (s, 3H), 3.59 (br. m, 1 H), 3.51 (br. m, 2H), 3.43 (br. m, 2H), 3.12 (m, 1 H), 2.98 (br. m, 5H), 2.86 (br. m, 2H), 2.73 (s, 3H), 1.96 (br. m, 2H), 1.82 (br. m, 2H), 1.72 (m, 2H), 0.90 (t, *J* = 7.2 Hz, 3H).
**1-Methyl-2-({4-[(4-methylpiperazin-1-yl)carbonyl]piperidin-1-yl}methyl)-1*H-*indole 1.3.1.1(15).** Yield is 56 %. LCMS (M+H)⁺ 355. ¹H NMR (DMSO-*d*₆, 400 MHz) δ 11.41 (br. s, 1 H), 10.93 (br. s, 1 H), 7.60 (d, *J* = 7.6 Hz, 1 H), 7.51 (d, *J* = 8.4 Hz, 1 H), 7.23 (m, 1 H), 7.08 (m, 1 H), 6.81 (s, 1 H), 4.54 (br. s, 2H), 4.40 (br. m, 1 H), 4.12 (br. m, 1 H), 3.85 (s, 3H), 3.50 (br. m, 5H), 3.00 (br. m, 4H), 2.87 (br. m, 2H), 2.73 (s, 3H), 1.98 (br. m, 2H), 1.82 (br. m, 2H).
**1-Methyl-2-({3-[(4-methylpiperazin-1-yl)carbonyl]piperidin-1-yl}methyl)-1*H-*indole dihydrochloride 1.3.2.1(1).** Yield is 46 %. LCMS (M+H)⁺ 355. ¹H NMR (DMSO-*d*₆, 400 MHz) δ 11.28 (br. s, 1 H), 11.14 (br. s, 1 H), 7.59 (d, *J* = 8.0 Hz, 1 H), 7.50 (d, *J* = 8.0 Hz, 1 H), 7.22 (m, 1 H), 7.07 (m, 1H), 6.83 (br. m, 1 H), 4.57 (br. s, 2H), 4.37 (br. m, 1 H), 4.07 (br. m, 1 H), 3.87 (s, 3H), 3.55 (br. m, 1 H), 3.42 (br. m, 4H), 3.14 (br. m, 2H), 3.02 (br. m, 3H), 2.89 (br. m, 1 H), 2.72 (br. m, 3H), 1.98 (br. m, 1 H), 1.86 (br. m, 2H), 1.44 (br. m, 1 H).
**1-Methyl-2-({3-[(4-propylpiperazin-1-yl)carbonyl]piperidin-1-ylmethyl)-1*H-*indole dihydrochloride 1.3.2.1(2).** Yield is 47 %. LCMS (M+H)⁺ 383. ¹H NMR (DMSO-*d*₆, 400 MHz) δ 11.19 (br. m, 2H), 7.59 (d, *J* = 7.6 Hz, 1 H), 7.50 (d, *J* = 8.0 Hz, 1 H), 7.22 (m, 1 H), 7.07 (m, 1 H), 6.83 (br. m, 1 H), 4.56 (br. s, 2H), 4.37 (m, 1 H), 4.05 (m, 1 H), 3.87 (s, 3H), 3.61 (m, 1 H), 3.44 (br. m, 5H), 3.14 (br. m, 2H), 2.98 (br. m, 4H), 2.86 (br. m, 1 H), 1.98 (br. m, 1 H), 1.86 (br. m, 2H), 1.70 (br. m, 2H), 1.44 (br. m, 1 H), 0.89 (br. m, 3H).
**1-(4-chlorobenzyl)-2-({3-[(4-methylpiperazin-1-yl)carbonyl]piperidin-1-yl}methyl)-1*H*-indole dihydrochloride 1.3.2.1(12).** Yield is 79 %. LCMS (M+H)⁺ 465, 467. ¹H NMR (DMSO-*d*₆, 400 MHz) δ 11.07 (br. m, 2H), 7.63 (d, *J* = 7.6 Hz, 1 H), 7.44 (d, *J* = 7.6 Hz, 1 H), 7.34 (d, *J* = 8.0 Hz, 2H), 7.17 (m, 1 H), 7.09 (m, 1 H), 6.98 (br. m, 1 H), 6.93 (d, *J* = 8.0 Hz, 2H), 5.69 (br. s, 2H), 4.50 (br. s, 2H), 4.38 (br. m, 1 H), 4.07 (br. m, 1 H), 3.49 (br. m, 5H), 3.16 (br. m, 2H), 3.00 (br. m, 3H), 2.89 (br. m, 1 H), 2.74 (br. m, 3H), 1.95 (br. m, 1 H), 1.86 (br. m, 2H), 1.43 (br. m, 1 H).
**1-(4-chlorobenzyl)-2-({3-[(4-propylpiperazin-1-yl)carbonyl]piperidin-1-yl}methyl)-1*H*-indole dihydrochloride 1.3.2.1 (13).** Yield is 78 %. LCMS (M+H)⁺ 493,495. ¹H NMR (DMSO-*d*₆, 400 MHz) δ 11.12 (br. m, 2H), 7.63 (d, *J* = 7.6 Hz, 1 H), 7.44 (d, *J* = 8.0 Hz, 1 H), 7.34 (d, *J* = 8.0 Hz, 2H), 7.17 (m, 1 H), 7.09 (m, 1 H), 6.99 (br. m, 1 H), 6.93 (d, *J* = 8.0 Hz, 2H), 5.69 (br. s, 2H), 4.51 (br. s, 2H), 4.37 (br. m, 1 H), 4.06 (br. m, 1 H), 3.59 (m, 1H), 3.41 (br. m, 5H), 3.14 (br. m, 2H), 2.98 (br. m, 4H), 2.85 (br. m, 1 H), 1.96 (br. m, 1 H), 1.86 (br. m, 2H), 1.70 (br. m, 2H), 1.43 (br. m, 1 H), 0.90 (br. m, 3H).
**1-Methyl-2-({3-[(4-propylpiperazin-1-yl)carbonyl]piperidin-1-yl}methyl-1*H-*indole dihydrochloride 1.3.2.1(14).** Yield is 47 %. LCMS (M+H)⁺ 383. ¹H NMR (DMSO-*d*₆, 400 MHz) δ 11.19 (br. m, 2H), 7.59 (d, J = 7.6 Hz, 1 H), 7.50 (d, J = 8.0 Hz, 1 H), 7.22 (m, 1 H), 7.07 (m, 1 H), 6.83 (br. m, 1H), 4.56 (br. s, 2H), 4.37 (m, 1 H), 4.05 (m, 1 H), 3.87 (s, 3H), 3.61 (m, 1 H), 3.44 (br. m, 5H), 3.14 (br. m, 2H), 2.98 (br. m, 4H), 2.86 (br. m, 1 H), 1.98 (br. m, 1 H), 1.86 (br. m, 2H), 1.70 (br. m, 2H), 1.44 (br. m, 1 H), 0.89 (br. m, 3H).

**Example 4.** Preparation of a medicament in the form of tablets. Starch (1600 mg), grained lactose (1600 mg), talcum (400 mg) and [1-(1-methyl-1*H*-indol-2-ylmethyl)-piperidin-4-yl]-(4-propyl-piperazin-1-yl)-methanone dihydrochloride 1.3.1.1(14) (1000 mg) were mixed together and pressed in a brick. Prepared brick was crushed to granules and riddled through sieves, gathering granules of 14-16 mesh size. The obtained granules were pelletised in tablets of suitable form of 560 mg by weight each.

**Example 5.** Preparation of a medicament in the form of capsules. [1-(1-Methyl-1*H-*indol-2-ylmethyl)-piperidin-4-yl]-(4-propyl-piperazin-1-yl)-methanone dihydrochloride **1.3.1.1(14)** was carefully mixed with lactose powder in ratio 2 : 1. The prepared powdery mixture was packed on 300 mg into gelatinous capsules of suitable size.

**Example 6.** Preparation of a medicament in the form of compositions for intramuscular, intraperitoneal or hypodermic injections. [1-(1-Methyl-1*H*-indol-2-ylmethyl)-piperidin-4-yl]-(4-propyl-piperazin-1-yl)-methanone dihydrochloride (500 mg) **1.3.1.1(14)** was dissolved in the mixture of chlorobutanole (300 mg), propylene glycol (2 ml), and water for injections (100 ml). The prepared solution was filtered and placed in 1 ml ampoules which were sealed up and sterilized in an autoclave.

### Industrial applicability

The invention could be used in medicine, veterinary, biochemistry.

## Claims

1. Antiviral active component, representing substituted indole of the general formula 1 and pharmaceutically acceptable salts thereof 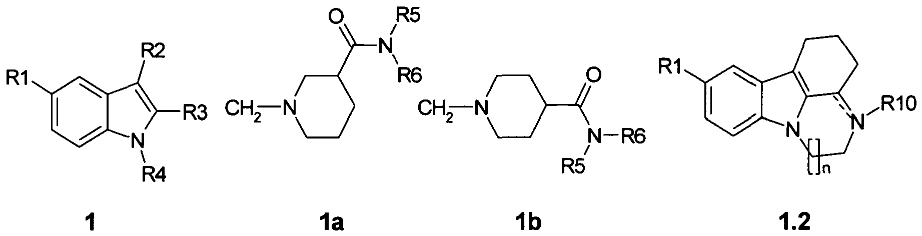 wherein:
**R1** represents hydrogen, optionally substituted C₁-C₄alkyl, C₆ cycloalkyl, aryl, ethoxycarbonyl, nitro group;
**R2** represents hydrogen;
**R3** represents N-mono- or N,N-di- substituted 1-methylene-piperidine-3-carboxamide of the general formula **1a** or N-mono- or *N,N*-di-substituted 1-methylene-piperidine-4-carboxamide of the general formula 1 b;
**R4** represents hydrogen, optionally substituted C₂-C₃ alkyl, R12-CH₂-group,
where
**R12** represents hydrogen or phenyl optionally substituted with C₁-C₄ alkyl or
halogen or
**R2, R3,** and **R4** together with the atoms they are attached to form substituted azaheterocycles of the general formula **1.2;**
**R5** and **R6** optionally identical represent hydrogen, optionally substituted C₁-C₃ alkyl or C₃-C₆ cycloalkyl, or
**R5** and **R6** together with the N-atom they are attached to form optionally substituted 5- or 6- membered azaheterocyclyl comprising one or two nitrogen atoms and optionally condensed with benzene ring, or form group 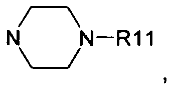
where:
**R11** represents hydrogen, C₁-C₆ alkyl, C₃-C₆ cycloalkyl;
**R10** represents hydrogen, optionally substituted C₁-C₃ alkyl; substituted acetyl; n = 1 or 2;
solid line accompanied by the dotted line, i.e. 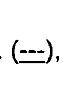 represents single or double C-C bond.

2. Antiviral active component according to claim 1, representing substituted amide of 1-[(1H-indol-2-yl)methyl]piperidine-4-carboxylic acid of the general formula 1.3.1 or substituted amide of 1-[(1H-indol-2-yl)methyl]piperidine-3-carboxylic acid of the general formula **1.3.2,** and pharmaceutically acceptable salts thereof 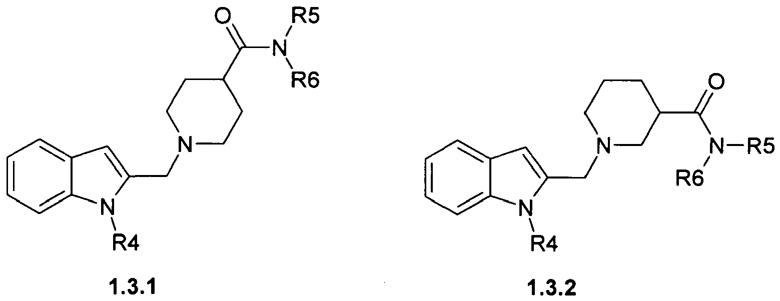 wherein:
**R4, R5** and **R6** have the above meanings.

3. Antiviral active component according to claim 1, representing substituted amide of 1-[(1*H*-indol-2-yl)methyl]piperidine-4-carboxylic acid of the general formula 1.3.1.1, and pharmaceutically acceptable salts thereof 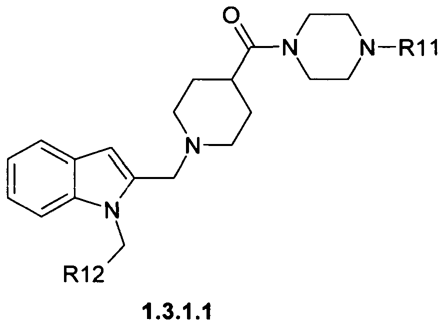 wherein:
**R11** and **R12** have the above meanings.

4. Antiviral active component according to claim 1, representing 2-(8-isopropyl-1,2,3a,4,5,6-hexahydro-3H-pyrazino[3,2,1-Jk]carbazol-3-yl)acetamide and its hydrochloride of formula **1.2(1)·HCl,**

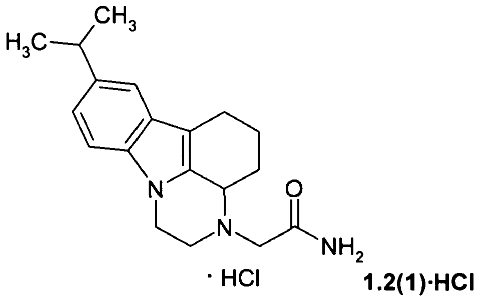

5. Antiviral component of the general formula 1.1, active towards hepatitis C virus (HCV), and pharmaceutically acceptable salts thereof 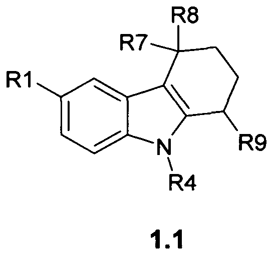 wherein:
**R1** represents methyl, ethoxycarbonyl, nitro group;
**R4** represents hydrogen, methyl, C₂-C₃ alkyl;
**R7** and **R8** represent hydrogen, or
**R7** and **R8** together with the C-atom they are attached to form C=O group;
**R9** represents azaheterocyclyl comprising at least one N-atom, unsubstituted formamide, or phenyl substituted with ethoxycarbonyl or nitro group

6. Pharmaceutical composition exhibiting antiviral activity and comprising pharmaceutically effective amount of an active component according to any of claims 1 - 4.

7. Pharmaceutical composition exhibiting antiviral activity towards hepatitis C virus (HCV and comprising pharmaceutically effective amount of an active component according to any of claims 1 - 5.

8. A method for preparation of pharmaceutical composition according to claim 7 by mixing at least one antiviral active component according to any of claims 1 - 5 with inert filler and/or solvent.

9. A medicament in the form of tablets, sheaths or injections placed in pharmaceutically acceptable packing for prophylaxis and treatment of viral diseases comprising an active component according to any of claims 1 - 4 or pharmaceutical composition according to claim 6 in effective amount.

10. A medicament in the form of tablets, sheaths or injections placed in pharmaceutically acceptable packing for prophylaxis and treatment of viral diseases caused by hepatisis C viruses (HCV), comprising an active component according to any of claims 1 - 5 or pharmaceutical composition according to claim 7 in effective amount.

11. A method for prophylaxis and treatment of viral diseases among them caused by hepatisis C viruses (HCV), by introduction to human of an active component according to any of claims 1 - 5, or pharmaceutical composition according to claims 6, 7, or medicament according to claims 9, 10 in effective amount.

12. A compound selected from the group representing substituted amides of 1-[(1H-indol-2-yl)methyl]piperidine-carboxylic acid of the general formula **1.3.2** or [1-(1-methyl-1*H*-indol-2-ylmethyl)-piperidin-4-yl]-(4-propylpiperazin-1-yl)-methanone **1.3.1.1 (14),** and pharmaceutically acceptable salts thereof, 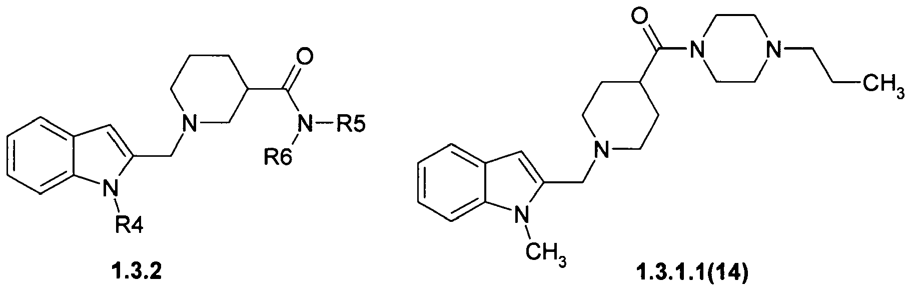 wherein:
**R4, R5** and **R6** have the above meanings.

13. A compound according to claim 12, representing substituted amide of 1-[(1 H-indol-2-yl)methyl]piperidine-carboxylic acid of the general formula **1.3.2.1,** and pharmaceutically acceptable salts thereof, 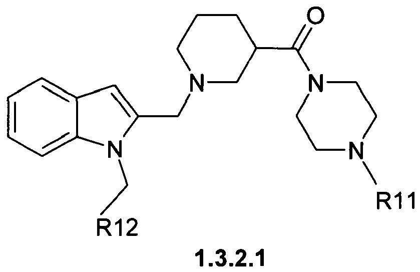 **R11** represents C₁-C₃alkyl; **R12** represents hydrogen or phenyl optionally substituted with halogen or C₁-C₃ alkyl.

14. A compound according to claim 13, selected from {1-[1-(4-chlorobenzyl)-1*H-*indol-2-ylmethyl]-piperidin-3-yl}-(4-propylpiperazin-1-yl)-methanone **1.3.2.1(13)** or [1-(1-methyl-1*H*-indol-2-ylmethyl)-piperidin-3-yl]-(4-propylpiperazin-1-yl)-methanone **1.3.2.1(14),** and hydrochloride thereof
